Europäisches Patentamt

**European Patent Office**

Office européen des brevets

(11) Veröffentlichungsnummer: **0 063 760**
A1

# EUROPÄISCHE PATENTANMELDUNG

(21) Anmeldenummer: 82103213.3

(22) Anmeldetag: 16.04.82

(51) Int. Cl.³: **G 01 B 11/30**, G 02 B 27/17, G 01 N 21/90

(30) Priorität: 18.04.81 DE 3115636

(43) Veröffentlichungstag der Anmeldung: 03.11.82 Patentblatt 82/44

(84) Benannte Vertragsstaaten: **AT BE CH FR GB IT LI LU NL SE**

(71) Anmelder: **Feldmühle Aktiengesellschaft, Fritz-Vomfelde-Platz 4, D-4000 Düsseldorf 11 (DE)**

(72) Erfinder: **Haubold, Wolfgang, Kollwitzstrasse 73, D-4800 Bielefeld 1 (DE)**

(74) Vertreter: **Uhlmann, Hans, Dr. rer.nat., Dipl.-Chem., Gladbacher Strasse 189, D-4060 Viersen 1 (DE)**

(54) Verfahren und Vorrichtung zum Prüfen der Innenflächen von runden Behältern.

(57) Das Prüfen der Innenflächen von runden Behältern, wie Getränkedosen, auf Oberflächen- und Lackierfehler, erfolgt mittels eines Lichtstrahls. Dieser Lichtstrahl wird auf einen rotierenden Spiegel geleitet und von diesem zu einem Abtastkreis abgelenkt. Der Abtastkreis hat im wesentlichen den gleichen Durchmesser wie der Behälterboden. Der Strahl wird vom Behälterboden auf die Behälterwandung reflektiert, das von diesem reflektierte Licht einem Fotoumwandler zugeleitet und die daraus entstehenden Impulse ausgewertet. Als Lichtstrahl wird ein Laserstrahl, der Strahlen in einer Wellenlänge zwischen 400 und 530 NM aussendet, verwertet. Der Strahl regt in der Lackschicht eine Fluoreszenz an. Durch Einschalten von Filtern zwischen Fotoumwandler und Prüfobjekt wird nur das fluoreszierende Licht ausgewertet.

Anmelder: Feldmühle AKtiengesellschaft,
Fritz-Vomfelde-Platz 4, 4ooo Düsseldorf 11

Verfahren und Vorrichtung zum Prüfen
der Innenflächen von runden Behältern.

Die Erfindung betrifft ein Verfahren und eine Vorrichtung zum Prüfen der Flächen von runden Behältern, wie Konserven- und Getränkedosen auf Oberflächenfehler, insbesondere fehlerhafte Innenlackierung.

Da Konserven- und Getränkedosen in größten Stückzahlen gefertigt werden, kommt der Einsatzmöglichkeit des Anmeldegegenstandes für diesen Einsatzzweck besondere Bedeutung zu, weshalb die Erfindung nachstehend als Beispiel an der Prüfung der Innenlackierung von Getränkedosen beschrieben wird, ohne sie jedoch hierauf zu beschränken.

Getränkedosen, insbesondere Dosen für Erfrischungsgetränke, wie kohlensäurehaltige Mineralwässer und Limonaden, aber auch Bier, werden in unterschiedliche Dosentypen abgefüllt. Bei einem Dosentyp handelt es sich um tiefgezogene Dosen mit nach innen gewölbten oder planen Böden; eine andere Dosenform entspricht der üblichen Konservendose, d.h. sie besteht aus einem gefalzten, ggf. geschweißten oder gelöteten Mantel mit angebördeltem Boden. Als Material für die Dosen wird im allgemeinen Weißblech eingesetzt; einige bestehen auch aus

- 2 -

Aluminiumblech. Allen Materialien ist jedoch gemeinsam, daß sie nicht mit Säuren, wie sie bei Getränken in Form von Kohlensäure oder Fruchtsäure vorhanden sind, in direkte Berührung kommen dürfen, da diese Stoffe das Metall angreifen und sich dadurch der Geschmack der abgepackten Ware verändert, die Ware dadurch verderben kann, aber auch die Verpackung beeinträchtigt wird. Der letztere Punkt kann so weit gehen, daß sich das verpackte Gut durch die Dose hindurchfrißt und durch Poren austritt.

Die Dosen werden, um solche Fehler und Beeinträchtigungen zu vermeiden, lackiert, was wegen der hohen anfallenden Stückzahl in Automaten erfolgt. Die Überprüfung der Dosen auf einwandfreie Lackierung erfolgt bisher im allgemeinen visuell beim Verpacken der Dosen. Eine absolute Gewähr, daß dabei jeder Lackierfehler erfaßt wird, ist nicht gegeben, da bei solchen Massenprodukten eine genaue visuelle Kontrolle, die ein Drehen der Dose um die eigene Achse und damit ein Betrachten aller Wandungsteile erfordert, zu aufwendig ist. Die alternative Möglichkeit zur Dosenherstellung besteht darin, zunächst das Blech zu beschichten und dieses beschichtete Blech dann tiefzuziehen. An die Beschichtung, also den Lack, werden dabei noch viel höhere Anforderungen gestellt, da er sich mit dem Blech verformen muß. Es treten folglich auch hier sowohl Blech- als auch Lackschäden auf, die erfaßt und aussortiert werden müssen.

Auf modernen Fertigungsstraßen fallen pro Aggregat 600 bis 1000 Stck. Dosen pro Minute an. Damit

- 3 -

steht zur Prüfung der einzelnen Dose nur der Bruchteil einer Sekunde zur Verfügung. Trotzdem soll innerhalb dieser kurzen Zeit ein eventuell vorhandener Fehler sicher ermittelt werden. Aufgabe der vorliegenden Erfindung ist es daher, eine Vorrichtung und ein Verfahren zu schaffen, die eine Prüfung großer Dosenmengen in kurzer Zeit nach objektiven Gesichtpunkten ermöglicht.

Diese Aufgabe wird gelöst durch ein Verfahren zum Prüfen der Flächen von runden Behältern, wie Konserven- und Getränkedosen auf Oberflächenfehler, insbesondere auf fehlerhafte Innenlackierung, mit dem kennzeichnenden Merkmal, daß ein Lichtstrahl auf einen rotierenden Spiegel geleitet, von diesem zu einem Abtastkreis geformt wird, wobei der Durchmesser des Abtastkreises im wesentlichen dem Durchmesser des Behälters entspricht, der Lichtstrahl vom Boden und/oder der Behälterwandung reflektiert und das zurückgeworfene Licht auf einen Fotoumwandler geleitet wird, der Impulse einer Auswertestation zuführt.

Durch den Einsatz eines Lichtstrahles und die Verwendung eines rotierenden Spiegels ergeben sich extrem hohe Abtastgeschwindigkeiten, wie sie für das Prüfen von Massengütern erforderlich sind. Durch den Spiegel wird ein rotierender Lichtpunkt geschaffen, der in der Prüfebene einen Abtastkreis bildet, wobei die Strichbreite dieses Abtastkreises vom Durchmesser des Lichtpunktes abhängig ist. Je feiner der Lichtpunkt, desto höher ist die Intensität, desto genauer können

- 4 -

damit auch kleine Fehler in der Beschichtung erfaßt werden. Wesentlich ist dabei für die Innenprüfung, daß der Abtastkreisdurchmesser geringfügig kleiner als der Innendurchmesser des Behälters ist, damit der Abtaststrahl nicht durch
Ablenkung an den Bodenkanten des Behälters Fehlersignale auslöst, ohne daß der Behälter einen
Fehler aufweist. Das aus dem Behälter zurückgeworfene Licht gelangt auf einen Fotoumwandler,
zweckmäßig einen Fotodetektor, der es in Impulse, entsprechend der Lichthelligkeit umwandelt und diese Impulse an eine Auswertestation
weitergibt.

Um eine möglichst hohe Lichtintensität zur Verfügung zu haben, sieht eine zweckmäßige Ausgestaltung der Erfindung vor, daß die Prüfung mit
einem Laserstrahl erfolgt. Laserstrahler senden
ein praktisch paralleles Licht aus, so daß keine
optischen Mittel zum Fokussieren eines Lichtpunktes erforderlich sind. Sie weisen auch über
ihre Lebensdauer hinweg eine praktisch konstante
Lichtstärke auf, so daß Aggregate zum Nachjustieren der Helligkeit entfallen können.

Eine bevorzugte Ausgestaltung der Erfindung sieht
vor, daß der Laserstrahler Strahlen einer Wellenlänge von 4oo bis 53o NM aussendet, d.h. in den
Farben blau und/oder grün strahlt. Diese Strahlung regt in der Lackschicht aller üblichen, zur
Innenlackierung von Behältern verwandten Lacke
eine Fluoreszenz an. Die gleiche Fluoreszenz
würde auch durch einen UV-Laser erreicht, jedoch
ist der Wirkungsgrad des UV-Lasers sehr gering,

- 5 -

so daß sein Einsatz Sonderzwecken vorbehalten
sein sollte.

Fluorszenz tritt selbstverständlich nur dort auf,
wo der Laserstrahl auf lackierte Flächen trifft.
In diesen Bereichen ergibt sich damit eine höhere
Lichtausbeute, so daß eine bessere Differenzierung zwischen fehlerhafter Stelle, d.h. unlackierter Stelle und fehlerfreier Stelle im Behälter
erfolgt. Die günstigste Auswertemöglichkeit ergibt sich jedoch, wenn gemäß einer besonders vorteilhaften Ausgestaltung der Erfindung nur das
fluoreszierende Licht ausgewertet wird. Dazu muß
der direkt reflektierte Teil der Laserstrahlung
abgefiltert werden, so daß nur der diffuse Lichtanteil in den Fotoumwandler fällt. In der praktischen Auswertung heißt das, daß eine nichtlackierte Dose überhaupt kein Lichtsignal erzeugt,
wohingegen teillackierte Dosen in den entsprechenden Bereichen keine Lichtsignale erzeugen. Da
der Lichtpunkt, der die Innenwandung des Behälters abtastet, sehr klein ist, werden bei der
Abtastung auch feine Fehler erfaßt, wie Poren
oder Luftblasen in der Lackierung, aber auch
Kratzer, die durch mechanische Beschädigung, beispielsweise beim Tiefziehen des Bleches, oder
im Anschluß an die Lackierung in die Behälter
eingebracht worden sind.

Eine zweckmäßige Ausgestaltung der Erfindung
sieht vor, daß die zu prüfenden Behälter kontinuierlich einer Prüfvorrichtung zugeführt und
unterhalb dieser in ihrer Position ausgerichtet
werden. Die Zuführung kann dabei durch eine üb-

- 6 -

liche Förderstrecke, beispielsweise in Form eines Förderbandes erfolgen, wobei zunächst eine Parallelausrichtung erfolgt, so daß die zu prüfenden Dosen in einer Reihe kontinuierlich unter der Prüfvorrichtung hergeführt werden. Durch Einsatz einer Lichtschranke ist es dabei möglich, ohne Unterbrechung der Fördergeschwindigkeit den Prüfkopf in dem Moment scharf zu machen, in dem ein zu prüfender Behälter die Prüfposition unter dem Meßkopf erreicht hat. Da bei der hohen Drehzahl von 30000 Umdrehungen des rotierenden Spiegels der Abtastzyklus nur 2 Millisekunden benötigt, also praktisch nur eine Momentaufnahme des in der Bewegung befindlichen Behälters stattfindet, stört die Behälterbewegung bei der Prüfung nicht, da sie auch bei Stückzahlen von 1000 Dosen pro Minute, verglichen mit der Abtastgeschwindigkeit, zu gering ist, um zu fehlerhaften Ergebnissen zu führen.

Eine bevorzugte Ausgestaltung der Erfindung sieht vor, daß der Kreisdurchmesser des Abtastkreises in seiner Größe wählbar ist. Diese Wählbarkeit ist von großer Bedeutung, weil Dosen mit den unterschiedlichsten Durchmessern anfallen, es aber zweckmäßig ist, mit ein und demselben Aggregat möglichst viele Durchmesser abtasten und damit überprüfen zu können. Die einfachste Möglichkeit zur Durchmesseränderung des Abtastkreises besteht in der Vergrößerung, bzw. Verkleinerung des Abstandes zwischen Spiegel und Behälter. Man wird diese Möglichkeit immer dann wählen, wenn keine zu großen Unterschiede in den Durchmessern vorhanden sind, die große Entfernungsunterschiede

- 7 -

bedingen und damit zu erheblichen Änderungen in
der Lichtintensität führen.

Im überwiegenden Teil aller Fälle ist es ausreichend, wenn bei dem zu prüfenden Behälter ein
ringförmiger Bereich des Bodens und ein ringförmiger Bereich des Dosenrumpfes geprüft wird.
Die Erklärung dafür ist im Lackiervorgang zu
suchen, der so vor sich geht, daß jede Dose um
die eigene Längsachse rotiert und sich bei dieser Rotation ein mit Düsen besetztes Spritzrohr
in die Dose einsenkt. Durch eine Düse wird dabei der Boden gespritzt, durch eine weitere der
Rumpf, also die Dosenwandung, so daß sich bei
diesem Lackiervorgang nur wenige Fehler ergeben
können. Der erste sich ergebende Fehler besteht
darin, daß der Düse kein Lack zugeführt wurde,
d.h., daß überhaupt keine Spritzung erfolgte.
Der zweite Fehler liegt in einer Düsenverstopfung.
Damit fällt entweder die Rumpf- oder die Bodenlackierung aus; und der dritte, mögliche Fehler,
die Dose rotierte nicht; dann ergibt sich nur
ein Lackstreifen auf Rumpf und Boden. Alle diese
Fehler können einwandfrei durch einen Abtastkreis, der auf dem Boden der Dose erzeugt und
von diesem auf den Rumpf reflektiert wird, erfaßt werden.

In den Fällen, in denen jedoch eine höhere Anforderung an das Prüfaggregat gestellt wird und eine
mehr oder weniger vollflächige Prüfung der Innenfläche einer Dose verlangt wird, müssen andere
Maßnahmen ergriffen werden. Eine vorteilhafte
Ausgestaltung der Erfindung sieht daher vor, daß

- 8 -

die Breite des Abtastkreises einstellbar ist.
Die Abtastung erfolgt daher zweckmäßig mit einem querverstreckten Lichtstrich. Je nach dem Verstreckungsgrad des Lichtstriches ist die Strichbreite des Abtastkreises mehr oder weniger groß, wobei dadurch, daß der Lichtstrich als solcher sehr schmal gehalten wird, auch die nötige Lichtintensität aufrechterhalten bleibt.

Eine bevorzugte Vorrichtung zur Durchführung des Verfahrens besteht im wesentlichen aus einer Förderstrecke mit darüber angeordnetem Meßkopf, einem Fotoumwandler und einer Auswertevorrichtung und weist das kennzeichnende Merkmal auf, daß der Meßkopf einen Strahler enthält, dem ein rotierender, schrägstehender Spiegel zugeordnet ist.

Als Strahler eignen sich punktförmige Lichtquellen, wie beispielsweise Halogenlampen. Zweckmäßig werden jedoch Laserstrahler eingesetzt, weil bei diesen bereits eine Bündelung des Strahles vorliegt, der im wesentlichen parallel austritt und dadurch keine Optik zur Bündelung und Erzeugung eines Lichtpunktes auf dem rotierenden Spiegel benötigt. Der rotierende Spiegel als solcher ist, weil er nur einen Lichtpunkt umleiten muß, relativ klein.

Die Drehzahl des rotierenden Spiegels beträgt gemäß einer zweckmäßigen Ausgestaltung der Erfindung 3ooo bis 30ooo Umdrehung pro Minute. Die hohen Geschwindigkeiten gestatten dabei, die Förderstrecke auch mit einer hohen Geschwindigkeit laufen zu lassen und damit große Stückzahlen

0063760

- 9 -

durchzusetzen, wie das für Massengüter gewünscht wird.

Eine bevorzugte Ausgestaltung der Erfindung sieht vor, daß die Stirnfläche der Rotorwelle eines Motors als schrägstehender Spiegel ausgeführt ist. Die Stirnfläche der Rotorwelle kann dabei direkt verspiegelt sein,es kann aber auch auf diese Fläche ein Spiegel aufgebracht werden, der dann in der jeweils erforderlichen Neigung zur Errei- chung einer bestimmten Abtastkreisgröße einge- stellt wird. Bei beiden Konstruktionen ist es erforderlich, daß der vom Strahler erzeugte Lichtpunkt über Spiegel oder Prismen auf den schrägstehenden Spiegel geleitet wird, ehe er von diesen zur Kreislinie abgelenkt werden kann.

Eine zweckmäßige alternative Lösung sieht vor, daß der rotierende Spiegel innerhalb eines, die Rotorwelle eines Motors bildenden Rohres angeord- net ist. Da der rotierende,schrägstehende Spiegel nur geringe Ausmaße aufweist, ergibt sich die Möglichkeit, ihn innerhalb eines Rohres mit kleinem Durchmesser anzuordnen und so den Aufwand für Prismen oder Spiegel zur Umleitung des Lichtpunktes auf den Spiegel zu vermeiden. Es genügt in diesem Falle, den Strahler direkt oberhalb des Motors anzuordnen, so daß er auf kürzestem Wege direkt in die Rotorwelle des Motors strahlt und den Lichtpunkt auf dem Spiegel abbildet.

Eine vorteilhafte Ausgestaltung der Erfindung sieht vor, daß der Motor von einem verspiegelten, durch eine lichtstreuende Scheibe abgeschlossenen

- 10 -

Raum umgeben ist, dem ein Fotoumwandler zugeordnet ist. Der Motor bildet dabei das zentrische Teil des Meßkopfes, durch den die Strahlung nach außen gelangt. Er ist, da er keine antreibende Funktion hat, sehr klein zu dimensionieren, so daß der ihn umgebende Raum im Meßkopf voll zur Aufnahme von Meßaggregaten zur Verfügung steht. Durch die Kombination, diesen Raum, der in umgibt, allseitig, ausgenommen die dem Prüfobjekt zugewandte Seite, zu verspiegeln und gleichzeitig durch eine lichtstreuende Scheibe abzuschließen, ist es möglich, das vom Prüfling direkt reflektierte Licht in diffuses Licht umzuwandeln, was die Erfassung von Fehlern auf hochglänzenden, spiegelnden Prüflingen ermöglicht und gleichzeitig auch diffus abgestrahltes Licht zu sammeln und dem Fotoumwandler zuzuführen. Durch die Vermeidung des Eintritts von direkt reflektiertem Licht tritt keine Überlastung des Fotoumwandlers auf, d.h., es werden Spitzen, die zu einer Überlastung des Aggregates führen könnten, vermieden.

Eine bevorzugte Ausgestaltung der Erfindung sieht vor, daß dem rotierenden Spiegel ein stationärer Spiegel zugeordnet ist, der ihn ringförmig umgibt. Zweckmäßig kann der stationäre Spiegel ein konischer Ring sein. Vorteilhaft ist auch die Ausführung als Parabolspiegel.

Sowohl die Ausführung als konischer Ring als auch als Parabolspiegel ermöglicht eine sehr einfache Veränderung des Abtastkreisdurchmessers. Durch einfache relative Höhenverstellung des stationären ringförmigen Spiegels zum rotierenden, schrägstehen-

- 11 -

den Spiegel, erfolgt eine Vergrößerung oder Verkleinerung des Abtastkreises, d.h., es ist ein sehr schneller Wechsel der Durchmesser möglich.

Von erheblicher Bedeutung ist die Anordnung eines stationären, ringförmigen Spiegels, jedoch nur aus dem weiteren Grund, daß mit einem so ausgerüsteten Aggregat durch Zuordnung eines weiteren stationären, ringförmigen Spiegels außer der Innenabtastung der Dosen, auch eine Außenabtastung des Dosenrumpfes erfolgen kann. Der konische Ring oder der Parabolspiegel ermöglichen die Erzeugung eines parallel zur Rotorwelle verlaufenden Abtaststrahles, der in diesem Falle auf einen weiteren, stationären Spiegelfeld und von diesem nahezu senkrecht auf den Mantelprüfling auftrifft. Ist der Strahl dabei bereits zu einem querverstreckten Lichtstrich verformt oder weist er die Form eines Zick-Zack-Bandes auf, wie er durch den Einsatz einer Vibrationsvorrichtung erzeugt werden kann, so können auch die Fehler auf der Außenseite des Rumpfes des Prüflings erfaßt werden.

Eine besonders bevorzugte Ausgestaltung der Erfindung sieht vor, daß dem Fotoumwandler ein Filter vorgeschaltet ist, der gemäß einer weiteren, zweckmäßigen Ausgestaltung der Erfindung für Wellenlängen unterhalb 53o NM undurchlässig ist. Diese Ausgestaltung der Erfindung ist besonders im Zusammenhang mit dem Einsatz eines Laserstrahles von Bedeutung, der in einer Wellenlänge von 4oo bis 53o NM strahlt. Bei dieser Wellenlänge handelt es sich um die Farben blau und/oder grün, die in der Lackschicht der Dose

0063760

- 12 -

eine Fluoreszenz anregen. Durch das Ausfiltern des eingestrahlten Lichtes wird nur das Licht vom Fotomultiplier erfaßt, das in der Lackschicht ein fluoreszierendes Licht, also ein Licht einer anderen Wellenlänge, im allgemeinen einer Wellenlänge von ca. 6oo NM umgewandelt wurde. Fehler in der Lackschicht führen damit zu sofortigem totalen Ausfall des Lichtpunktes, geben also ein sehr starkes Fehlersignal, wodurch eine feine Fehlerdifferenzierung möglich ist.

Um die Lichtintensität des fliegenden Lichtpunktes, der auf Grund der Rotation des Spiegels den Abtastring bildet, möglichst hoch zu belassen, weil durch die hohe Lichtintensität eine gute Auswertbarkeit auch kleiner Fehler gegeben ist, sieht eine zweckmäßige Ausgestaltung der Erfindung vor, daß der Spiegel in seiner Winkelstellung im Rohr verstellbar ist. Durch die Verstellbarkeit des Spiegels kann mit relativ einfachen Mitteln der Durchmesser des Abtastringes geändert werden, ohne daß eine wesentliche Verlängerung des Strahlenweges erfolgt, d.h., ohne daß wesentliche Lichteinbußen auftreten.

Aus dem gleichen Grund, also der Erhaltung der Lichtintensität, wird gemäß einer vorteilhaften Ausgestaltung der Erfindung für die Erzeugung einer größeren Strichbreite des Abtastkreises vorgeschlagen, den Spiegel mit einer Vibrationsvorrichtung zu versehen. Der von dem Laserstrahler erzeugte, fliegende Lichtstrich wird jetzt also einmal durch das Rotieren um seine Achse gedreht, zum anderen durch den beweglich mon-

- 13 -

tierten Spiegel über die Vibrationsvorrichtung um einen bestimmten Betrag auf- und abbewegt, so daß sich statt einer kreisförmigen Abtastung, eine Abtastung in Form eines Zick-Zack-Bandes ergibt, das ebenfalls Kreisform aufweist. Entsprechend der Frequenz der Vibrationsvorrichtung liegen die Zick-Zack-Strahlen dicht beieinander. Sie können sich dabei überschneiden oder, falls das für den gewünschten Anwendungszweck günstiger ist, in einem geringen Abstand nebeneinander verlaufen, wobei durch diesen Abstand gleichzeitig die Größe des Fehlers vorgegeben ist, der noch für den Anwendungszweck akzeptiert werden kann. Zweckmäßig liegt dabei die Frequenz, mit der der Spiegel vibriert, bei dem 10- bis 100-fachen der Drehzahlfrequenz.

Eine weitere vorteilhafte Ausgestaltung der Erfindung sieht vor, daß dem rotierenden Rohr ein durchbohrter Spiegel nachgeschaltet ist, dem zweckmäßig auf dem Weg zum zu prüfenden Behälter eine Lochblende folgt. Der durchbohrte Spiegel steht dabei unter einem schrägen Winkel zum zu prüfenden Behälter und ermöglicht dem durch den rotierenden Spiegel abgelenkten Strahl das Durchtreten durch die Bohrung auf den Behälter. Das von diesem, bzw. dem Dosenrumpf reflektierte Licht fällt auf Grund der Ablenkung auf den Spiegel und wird von diesem in Richtung des Fotoumwandlers, ggf. unter Zwischenschaltung einer Sammellinse und eines Filters, abgelenkt. Der Einsatz einer Lochblende zwischen durchbohrtem Spiegel und zu prüfendem Behälter ermöglicht, daß lediglich das diffus reflektierte Licht ausgewertet wird, das

- 14 -

direkt/reflektierte also ausgeblendet werden kann. Diese Vorrichtung ist besonders bei stark spiegelndem Material von Bedeutung, weil dadurch vermieden wird, daß der Fotoumwandler zu große Ähnlichkeitsunterschiede aufnehmen muß.

Die Erfindung wird nachstehend an Hand der Zeichnungen beschrieben.

Fig. 1 zeigt die Prüfvorrichtung im Teilschnitt von der Seite,

Fig. 2 die Vorderansicht der Prüfvorrichtung,

Fig. 3 Details aus dem Prüfkopf und

Fig. 4 einen Prüfkopf mit eingebautem konischen Ringspiegel.

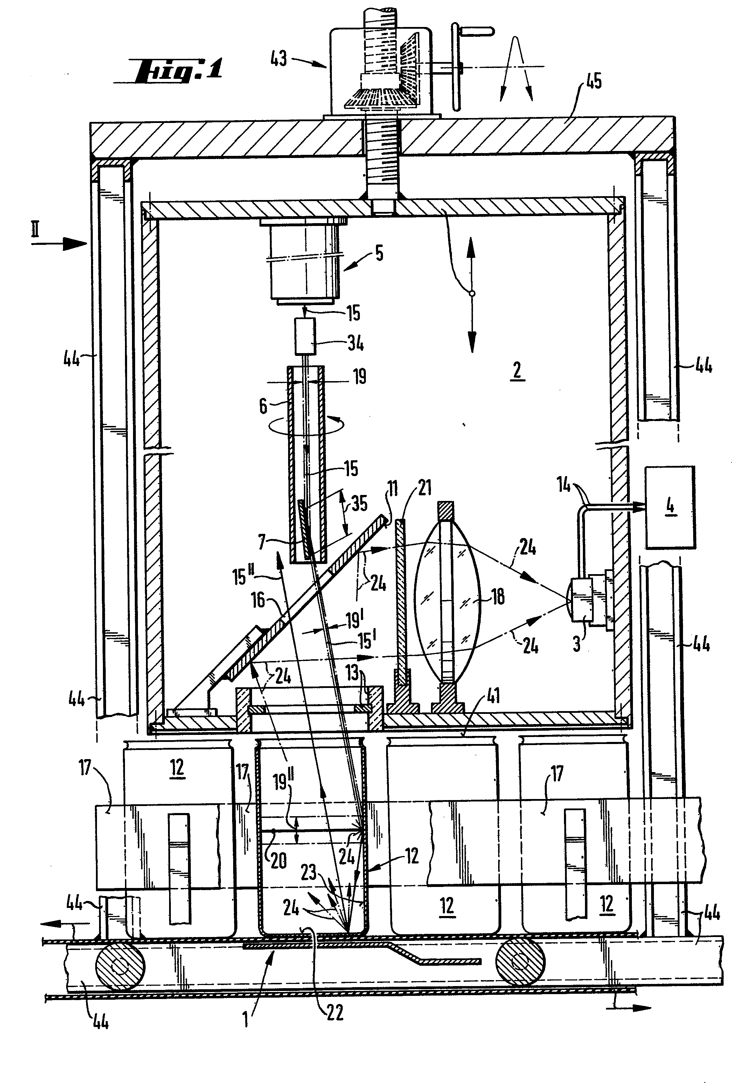

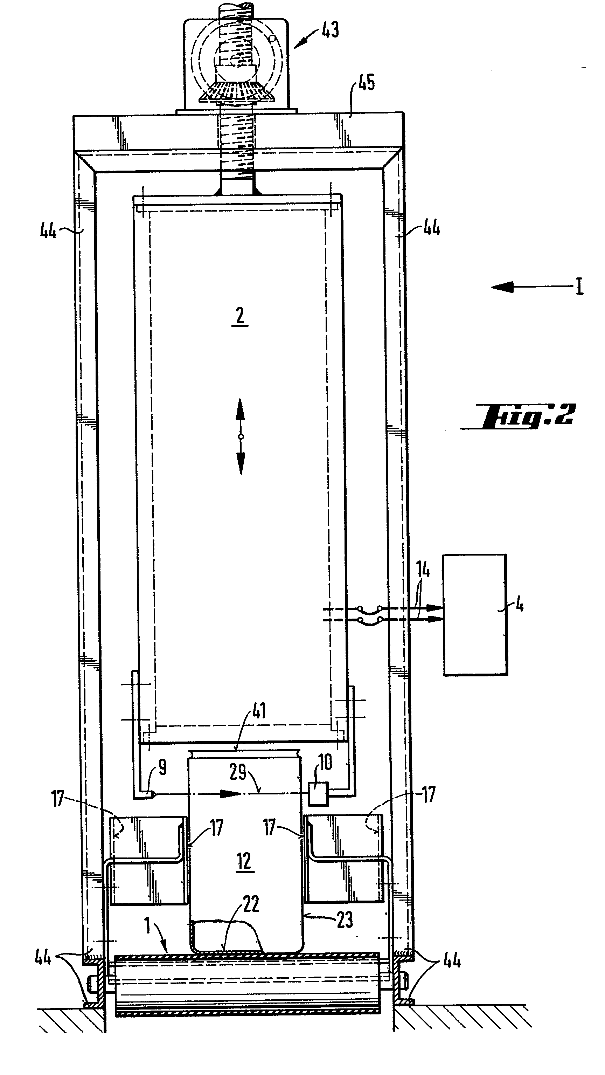

Auf einer Förderstrecke 1, gem.Fig 1 und 2, wird der Prüfling 12 unter dem Meßkopf 2 geführt,der über die Leitung 14 mit der Auswerteeinrichtung 4 verbunden ist. Im Meßkopf 2 ist der Strahler 5 angeordnet, der als Laserstrahler ausgeführt ist und einen Strahl 15 auf den schrägstehenden Spiegel 7 im rotierenden Rohr 6 wirft. Der Strahl 15 wird vom schrägstehenden Spiegel 7 als reflektierter Strahl 15' abgeleitet und tritt durch die Bohrung 16 des durchbohrten Spiegels 11. Nach Passieren der Lochblende 13 fällt der reflektierte Strahl 15' auf den Prüfling 12, der durch seitliche Führungen 17, die an der Förderstrecke 1 angeordnet sind, in den Abtastbereich geführt wird. Im Prüfling 12 trifft der Strahl 15' auf den Rumpf 23 der Dose auf und erzeugt durch den Umlauf des rotierenden Rohres 6 samt Spiegel 7 die Abtastlinie 2o. Gleichzeitig wird der Strahl 15' auf den Boden 22 des Prüf-lings 12 abgelenkt und von diesem als direktreflektierter Strahl 15'' durch die Bohrung 16 im durchbohrten Spiegel 11 aus dem Prüfling 12 zurückgeworfen. Bei einem Auftreffen auf den Rumpf 23 und den Boden 22 des Prüflings 12 wurde der Lack zu einer diffusen Strahlung 24 angeregt, die vom durchbohrten Spiegel 11 über den Filter 21 und die Sammellinse 18 auf den Fotoumwandler 3 gelangt. Von dort führt eine Leitung 14 zur Auswerteeinrichtung 4. Das evtl. übrige, direkt reflektierte Licht wird im wesentlichen von der Lochblende 13 ausgeblendet, evtl. doch noch auf den durchbohrten Spiegel 11 gelangende Strahlung durch den Filter 21 abgefildert.

Dem Strahler 5 kann eine Optik 34, z.B. aus einer Zerstreuungs- und einer Zylinderlinse, nachgeschaltet sein. Diese Optik 34 verstreckt den Strahl 15 quer zu seiner Strahllaufrichtung, so daß aus dem im Querschnitt punktförmigen Strahl 15 ein im Querschnitt strichförmiger Lichtstrahl 19 bzw. Lichtstrahlebene 19 entsteht. Dieser Lichtstrahl 19 erzeugt bei seinem Auftreffen auf den rotierenden Schrägspiegel 7 einen reflektierenden Lichtstrich 35, welcher als Parallelstrahlenebene 19' auf den Prüfling, hier in Fig.1 auf den Rumpf 23 trifft und dort eine zylinderringförmige Lichtprüffläche 19'' erzeugt. Diese Lichtprüffläche kann durch entsprechende Neigung des Spiegels 7 so groß eingestellt werden, daß sie z.B. die ganze Innenfläche des Rumpfes 23 bestreicht und/oder auch den Boden 22. Somit kann die ganze Prüffläche abgetastet werden.

- 16 -

Der Meßkopf ist, wie Fig. 2 zeigt, mit einer Lichtschranke 29 ausgerüstet, die aus dem Sender 9 und
dem Empfänger lo besteht. Die Lichtschranke 29 sendet Impulse zur Auswertestation 4, die ein Zählen
der geprüften Dosen ermöglichen. Die Auswertestation gibt bei Ausschuß Aussortierungsorder einer
der Prüfvorrichtung nachgeschalteten Auswerteeinheit. Der Meßkopf 2 ist an einem Vertialverstellantrieb 43 aufgehängt. Der Vertikalverstellantrieb
ruht auf einer Traverse 45, die wiederum das Haupt
eines Gestelles 44 bildet.

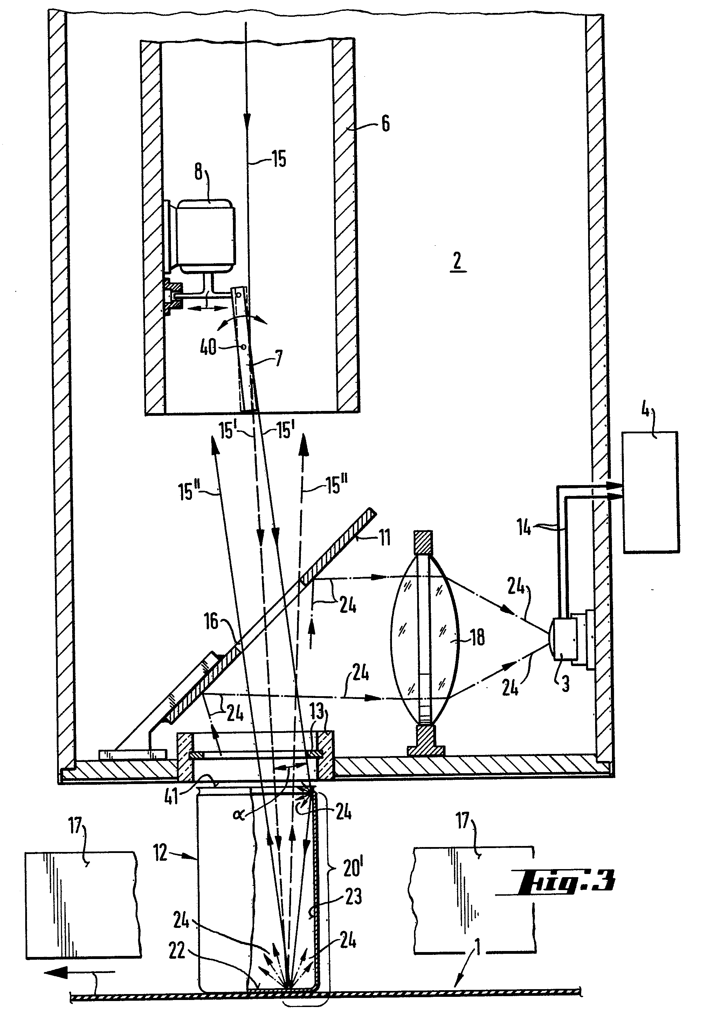

Fig. 3 zeigt, daß der schrägstehende Spiegel 7 gelenkig bei 4o im rotierenden Rohr 6 angeordnet ist
und von einer Vibrationsvorrichtung 8 beaufschlagt
wird, die dem Strahl 15' eine zusätzliche Bewegung,
wie durch Winkel $\alpha$ gekennzeichnet, verleiht. Außer
der vom rotierenden Rohr 6 herrührenden Kreisbewegung, legt der Strahl 15' jetzt zusätzlich eine
radiale Bewegung zurück, d.h., daß er statt der
lichtpunktstarken Abtastlinie 2o eine breite Abtastlinie 2o' beschreibt, die, wie Fig.3 zeigt,
den Bereich zwischen der Mitte des Bodens 22 des
Prüflings und dessen oberen Rand 41 am Rumpf 23
abdeckt. Der Prüfling 12 wird dadurch innen lückenlos abgetastet.

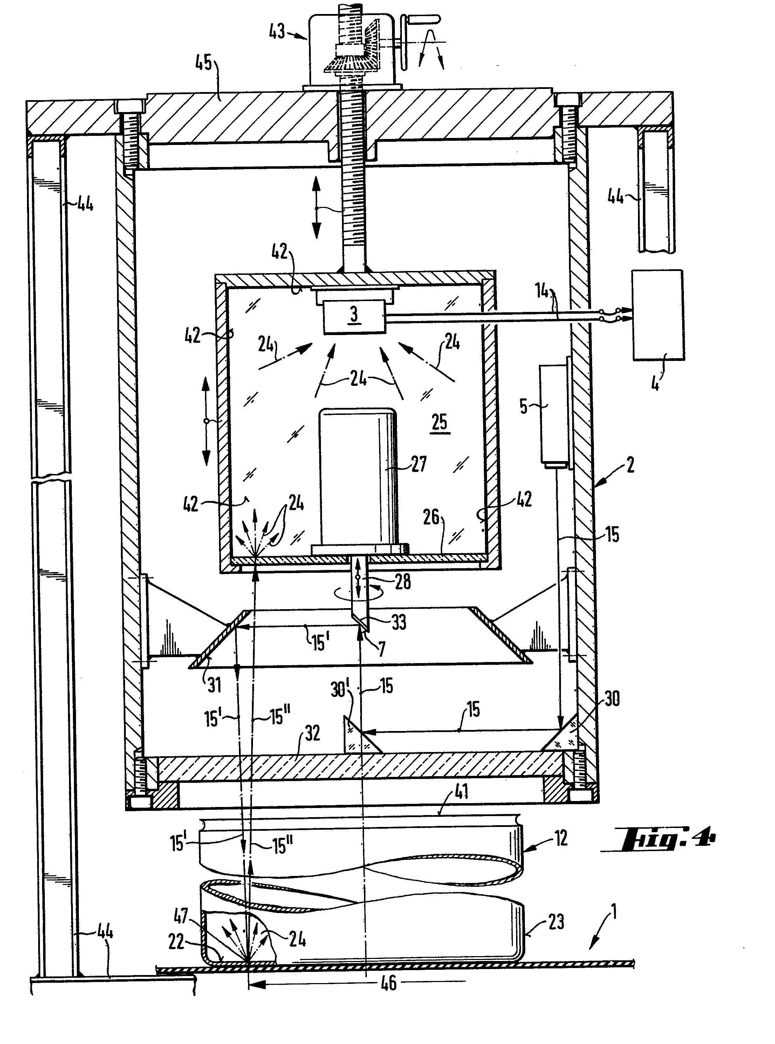

Fig.4 zeigt, daß der Fotoumwandler 3 in einem allseits an seinen Innenflächen 42 verspiegelten Raum
25 angeordnet ist, der von einer lichtstreuenden
Scheibe 26 abgedeckt ist. In dem verspiegelten
Raum 25 befindet sich auch der Motor 27, dessen
Rotorwelle 28 aus dem Raum 25 durch die lichtstreuende Scheibe 26 herausragt. Die Stirnfläche 33 der Motorwelle 28 ist abgeschrägt und bildet oder trägt den schrägstehenden Spiegel 7. Durch den im Meßkopf 2 innen angeordneten Strahler 5 wird ein Strahl 15 auf ein Prisma 3o geworfen, von hier auf ein zweites Prisma 3o' abgelenkt und von diesem auf den schrägstehenden Spiegel 7 geleitet. Der vom schrägstehenden Spiegel 7 reflektierte Strahl 15' fällt auf den stationären Spiegel 31, der die Rotorwelle 28 konusringförmig umgibt und wird von diesem Spiegel 31 auf den Prüfling 12 abgelenkt und bildet dort die Abtastlinie 47. Er passiert dabei die Abdeckscheibe 32, die den Meßkopf 3 nach unten verschließt. Durch den Vertikal-Stell-Antrieb kann nun der Spiegel 7 relativ zum Ringspiegel 31 verstellt werden. Dadurch ist eine Einstellung des Durchmessers 46 der Abtastlinie 47 gegeben.

- 18 -

Anmelder: Feldmühle Aktiengesellschaft,
Fritz-Vomfelde-Platz 4, 4ooo Düsseldorf 11

Patentansprüche.

1. Verfahren zum Prüfen der Flächen von runden Behältern, wie Konserven- und Getränkedosen auf Oberflächenfehler, insbesondere fehlerhafte Innenlackierung, dadurch gekennzeichnet, daß ein Lichtstrahl auf einen rotierenden Spiegel geleitet, von diesem zu einem Abtastkreis abgelenkt wird, wobei der Abtastkreis im wesentlichen gleich dem Durchmesser des Behälters ist, der Strahl vom Boden und/oder der Behälterwandung reflektiert und das zurückgeworfene Licht auf einen Fotoumwandler geleitet wird, der daraus resultierende Impulse einer Auswertestation zuführt.

2. Verfahren nach Anspruch 1, dadurch gekennzeichnet, daß die Prüfung mit einem Laserstrahl erfolgt.

3. Verfahren nach einem der Ansprüche 1 und 2, dadurch gekennzeichnet, daß der Laser Strahlen in den Wellenlängen zwischen 4oo und 53o Nanometer aussendet.

4. Verfahren nach einem der Ansprüche 1 bis 3, dadurch gekennzeichnet, daß der Laserstrahl in der Lackschicht eine Fluoreszenz anregt.

5. Verfahren nach einem der Ansprüche 1 bis 4, dadurch gekennzeichnet, daß das fluoreszierende Licht ausgewertet wird.

6. Verfahren nach einem der Ansprüche 1 bis 5, dadurch gekennzeichnet, daß die Behälter kontinuierlich einer Prüfvorrichtung zugeführt und unterhalb dieser in ihrer Position ausgerichtet werden.

7. Verfahren nach einem der Ansprüche 1 bis 6, dadurch gekennzeichnet, daß der Durchmesser des Abtastkreises in seiner Größe wählbar ist.

8. Verfahren nach einem der Ansprüche 1 bis 7, dadurch gekennzeichnet, daß die Strichbreite des Abtastkreises einstellbar ist.

9. Verfahren nach einem der Ansprüche 1 bis 8, dadurch gekennzeichnet, daß die Abtastung mit einem querverstreckten Lichtstrich (19/35/19'/19'') erfolgt.

lo. Vorrichtung zur Durchführung des Verfahrens nach einem der Ansprüche 1 bis 9, im wesentlichen bestehend aus einer Förderstrecke (1) mit darüber angeordnetem Meßkopf (2), einem Fotoumwandler (3) und einer Auswertevorrichtung (4), dadurch gekennzeichnet, daß der Meßkopf (2) einen Strahler (5) enthält, dem ein rotierender, schrägstehender Spiegel (7) zugeordnet ist.

11. Vorrichtung nach Anspruch lo, dadurch gekennzeichnet, daß die Drehzahl des rotierenden Spiegels (7) 3ooo bis 3oooo Umdrehungen/Min. beträgt.

12. Vorrichtung nach einem der Ansprüche lo und 11, dadurch gekennzeichnet, daß der rotierende Spiegel (7) innerhalb eines, die Rotorwelle (28) eines Motors (27) bildenden Rohres (6) angeordnet ist.

13. Vorrichtung nach einem der Ansprüche lo und 11, dadurch gekennzeichnet, daß die Stirnfläche (33) der Rotorwelle (28) als schrägstehender Spiegel (7) ausgeführt ist, bzw. einen solchen trägt.

14. Vorrichtung nach einem der Ansprüche lo bis 13, dadurch gekennzeichnet, daß der Motor (27) von einem verspiegelten, durch eine lichtstr-euende Scheibe (26) abgeschlossenen Raum (25) umgeben ist, dem ein Fotoumwandler (3) zugeordnet ist.

15. Vorrichtung nach einem der Ansprüche lo bis 14, dadurch gekennzeichnet, daß dem rotierenden Spiegel (7) ein stationärer Spiegel (31) zugeordnet ist, der ihn ringförmig umgibt.

16. Vorrichtung nach einem der Ansprüche lo bis 15, dadurch gekennzeichnet, daß der stationäre Spiegel (31) ein konischer Ring ist.

17. Vorrichtung nach einem der Ansprüche lo bis 16, dadurch gekennzeichnet, daß der stationäre Spiegel (31) ein Parabolspiegel ist.

18. Vorrichtung nach einem der Ansprüche lo bis 17, dadurch gekennzeichnet, daß dem Fotoumwandler (3) ein Filter (21) vorgeschaltet ist.

19. Vorrichtung nach einem der Ansprüche lo bis 18, dadurch gekennzeichnet, daß der Filter (21) für Wellenlängen kleiner als 53o NM undurchlässig ist.

2o. Vorrichtung nach einem der Ansprüche lo bis 19, dadurch gekennzeichnet, daß der schrägstehende Spiegel (7) in seiner Winkelstellung um ein Gelenk (4o) verstellbar ist.

21. Vorrichtung nach einem der Ansprüche lo bis 2o, dadurch gekennzeichnet, daß der schrägstehende Spiegel (7) mit einer Vibrationsvorrichtung (8) versehen ist.

22. Vorrichtung nach einem der Ansprüche lo bis 21 dadurch gekennzeichnet, daß dem Strahler (5) eine Optik (34) zur Querverstreckung des Strahles (15) nachgeschaltet ist.

23. Vorrichtung nach einem der Ansprüche lo bis 22, dadurch gekennzeichnet, daß dem rotierenden Rohr (6) ein durchbohrter Spiegel (11) nachgeschaltet ist.

24. Vorrichtung nach einem der Ansprüche lo bis 23, dadurch gekennzeichnet, daß zwischen dem durchbohrten Spiegel (11) und dem zu prüfenden Behälter (12) eine Lochblende (13) angeordnet ist.

0063760

**Fig.1**

**_Fig.2_**

**Fig:3**

0063760

Fig. 4

Europäisches
Patentamt

**EUROPÄISCHER RECHERCHENBERICHT**

0063760

Nummer der Anmeldung

EP 82 10 3213.3

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| A | DE – B – 1 648 640 (B.J. O'CONNOR et al.)  * Ansprüche; Spalte 3; Fig. 1 *  -- | 1,10 |
| A | DE – A1 – 2 910 516 (KIRIN BEER)  * Ansprüche 1, 2, 8, 11 *  -- | 1,2,10 |
| A | US – A – 3 150 266 (B.B. MATHIAS)  * Spalten 1, 2; Fig. 1, 3 *  ---- | 1,10 |

**KLASSIFIKATION DER ANMELDUNG (Int Cl.)**

G 01 B 11/30

G 02 B 27/17

G 01 N 21/90

**RECHERCHIERTE SACHGEBIETE (Int. Cl.)**

G 01 B 11/00

G 01 N 21/00

G 02 B 27/17

**KATEGORIE DER GENANNTEN DOKUMENTE**

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument

&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Berlin | 26-07-1982 | KÖHN |

EPA form 1503.1   06.78